# EUROPEAN PATENT APPLICATION

(11) **EP 3 284 414 A1**
(43) Date of publication of application: **21.02.2018**
(21) Application number: 16779820.6
(22) Date of filing: 24.02.2016
(51) Int. Cl.: A61B 17/11

(54) **ANASTOMOSIS CONNECTOR**

(30) Priority: 17.04.2015 JP 2015085399
(71) Applicant: Jichi Medical University, Tochigi 329-0498 (JP); Shizuoka Prefecture, Shizuoka-shi Shizuoka 420-8601 (JP); Tamachi Industries Co., Ltd., Shinagawa-ku, Tokyo 140-0013 (JP)
(72) Inventor: SARUKAWA Shunji, Shimotsuke-shi Tochigi 329-0498 (JP); KAMOCHI Hideaki, Shimotsuke-shi Tochigi 329-0498 (JP); INOUE Keita, Sunto-gun Shizuoka 411-8777 (JP); SAEGUSA Noriko, Sunto-gun Shizuoka 411-8777 (JP); NAKAGAWA Masahiro, Sunto-gun Shizuoka 411-8777 (JP); OHTA Kunihiro, Tokyo 140-0013 (JP)
(74) Representative: Betten & Resch
(86) International application number: PCT/JP2016/055384
(87) International publication number: WO 2016/167028

(57) **Abstract**

An anastomosis device 3 includes a corrugated circumference section (20;120;120'), a top-side slender member (10A-1;110A-1), and a bottom-side slender member (10B-1;110B-12). The top-side slender member (10A-1;110A-1) is connected to one top section (20A-1;30A-1;120A-1) of the corrugated circumference section (20; 120;120'). The bottom-side slender member (10B-1;110B-12) is connected to one (20B-1;30B-1;120B-12) at the corrugated circumference section (20;120;120').

## Description

### Technical Field

The present invention relates to an anastomosis device.

### Background Art

When a first tubular biological tissue and a second tubular biological tissue are inosculated, an anastomosis device is sometimes used.

In Patent Literature 1, a technique is disclosed in which the inosculation is assisted by inserting a first end of the anastomosis device into the first tubular tissue, and a second end of the anastomosis device into the second tubular tissue (especially, with reference to FIG. 36A to FIG. 36G in Patent Literature 1)

### Citation List

[Patent Literature 1] WO 2009/126244

### Summary of the Invention

An object of the present invention is to provide an anastomosis device that is easy to insert in a tubular tissue.

The anastomosis device according to some embodiments is an anastomosis device which combines a first tubular body and a second tubular body. The anastomosis device includes: a corrugated circumference section having a plurality of top sections and a plurality of bottom sections, which are alternately arranged; a top-side slender member; and a bottom-side slender member. The top-side slender member includes a first proximal end connected to one of the plurality of top sections; and a first free end engageable with the first tubular body. The top-side slender member protrudes from the one top section to a first direction. The bottom-side slender member includes a second proximal end connected to one of the plurality of bottom sections, and a second free end engageable with the second tubular body. The bottom-side slender member protrudes from the one bottom section to a second direction which is different from the first direction.

An anastomosis device of some embodiments includes a corrugated circumference section having a plurality of top sections and a plurality of bottom sections, which are alternately arranged; a plurality of top-side slender members; and a plurality of bottom-side slender members. Each of the plurality of top-side slender members has a first proximal end connected to one of the plurality of top sections, and a first free end. Each of the plurality of bottom-side slender members has a second proximal end connected to one of the plurality of bottom sections, and a second free end.

According to the present invention, the anastomosis device can be provided that is easy to insert into a tubular tissue.

### Brief Description of the Drawings

The attached drawings are incorporated into this Specification to help the description of embodiments. Note that the drawings should not be interpreted to limit the present invention to illustrated or described examples.
[FIG. 1A] FIG. 1A is a diagram to describe an inosculation technique.
[FIG. 1B] FIG. 1B is a diagram to describe the inosculation technique.
[FIG. 1C] FIG. 1C is a diagram to describe the inosculation technique.
[FIG. 1D] FIG. 1D is a diagram to describe the inosculation technique.
[FIG. 2] FIG. 2 is a diagram to describe a contraction state of an anastomosis device.
[FIG. 3] FIG. 3 is a schematic perspective view of the anastomosis device in an embodiment.
[FIG. 4] FIG. 4 is a schematic perspective view of the anastomosis device in an embodiment.
[FIG. 5] FIG. 5 is a schematic perspective view of the anastomosis device in an embodiment.
[FIG. 6] FIG. 6 is a development view of the anastomosis device in an embodiment.
[FIG. 7] FIG. 7 is a development view of a part of the anastomosis device in an embodiment.
[FIG. 8] FIG. 8 is a sectional view when viewed from a plane along the A-A arrow in FIG. 7.
[FIG. 9] FIG. 9 is a diagram to describe an effect by a corrugated circumference section.
[FIG. 10] FIG. 10 is a diagram to describe the effect by the corrugated circumference section.
[FIG. 11] FIG. 11 is a diagram to describe the effect by the corrugated circumference section.
[FIG. 12] FIG. 12 is a diagram to describe the effect by the corrugated circumference section.
[FIG. 13] FIG. 13 is a diagram showing a modification example of a first free end and a second free end of a slender member.
[FIG. 14] FIG. 14 is a diagram showing a modification example of the corrugated circumference section.
[FIG. 15] FIG. 15 is a development view of a part of the anastomosis device in an embodiment.
[FIG. 16] FIG. 16 is a development view of the anastomosis device in the embodiment.
[FIG. 17] FIG. 17 is an embodiment development of a part of the anastomosis device.
[FIG. 18] FIG. 18 is a development view of the anastomosis device in the embodiment
[FIG. 19] FIG. 19 is a development view of the corrugated circumference section.
[FIG. 20] FIG. 20 is a development view of a part of the anastomosis device.
[FIG. 21] FIG. 21 is a schematic side view of the anastomosis device.
[FIG. 22] FIG. 22 is a development view of the anastomosis device in the embodiment.
[FIG. 23] FIG. 23 is a development view of the corrugated circumference section.
[FIG. 24] FIG. 24 is a development view of a part of the anastomosis device.
[FIG. 25] FIG. 25 is a schematic side view of the anastomosis device.

### Description of the Embodiments

Hereinafter, with reference to the attached drawings, the description of the embodiments is carried out. In the following detailed description, many detailed specific items are disclosed for the purpose of the description to provide the comprehensive understanding of the embodiments. However, it would be clear that one or a plurality of embodiments are executable without these detailed specific items.

### (Definition of terms)

In this Specification, the term of "outer diameter" in a fixed cross section of a member is defined as the maximum value of distance between one point on the outer circumference of the member and another point on the outer circumference of the member in the fixed cross section of the member.

In this Specification, a "straight line" includes a substantially straight line in addition to a fully straight line.

### (Explanation of inosculation technique)

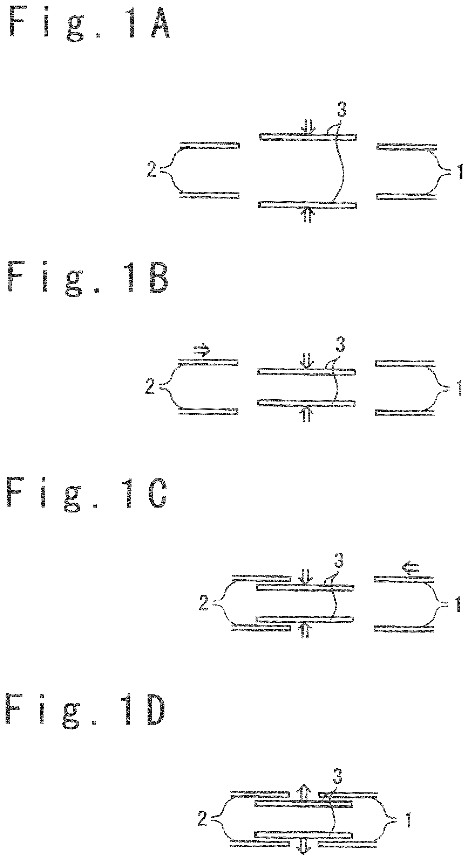

Referring to FIG. 1A to FIG. 1D, an inosculation technique will be described. FIG. 1A shows a first process of the inosculation technique, FIG. 1B shows a second process thereof, FIG. 1C shows a third process thereof, and FIG. 1D shows a fourth process thereof.

FIG. 1A is a sectional view of a first tubular body 1 which is a biological tissue, a second tubular body 2 which is a biological tissue, and a anastomosis device 3. The anastomosis device 3 is a member that has an annular part. In the first process, a central section of the anastomosis device 3 in a longitudinal direction is supported by a holding tool (not shown). In the first process, the central section of the anastomosis device 3 in the longitudinal direction is contracted by the holding tool.

In the second process shown in FIG. 1B, the anastomosis device 3 is inserted into the second tubular body 2 in a contraction state held by the holding tool.

In the third process shown in FIG. 1C, the anastomosis device 3 is inserted in the first tubular body 1 in the contraction state held by the holding tool. Note that in an example shown in FIG. 1B and FIG. 1C, the second process is carried out previously to the third process. Alternatively, the third process (the insertion into the first tubular body 1) may be carried out previously to the second process (the insertion into the second tubular body 2).

In the fourth process shown in FIG. 1D, the anastomosis device 3 is released from the holding tool to be expanded. Thus, the anastomosis device 3 is fixed on an inner wall of the first tubular body 1 and an inner wall of the second tubular body 2.

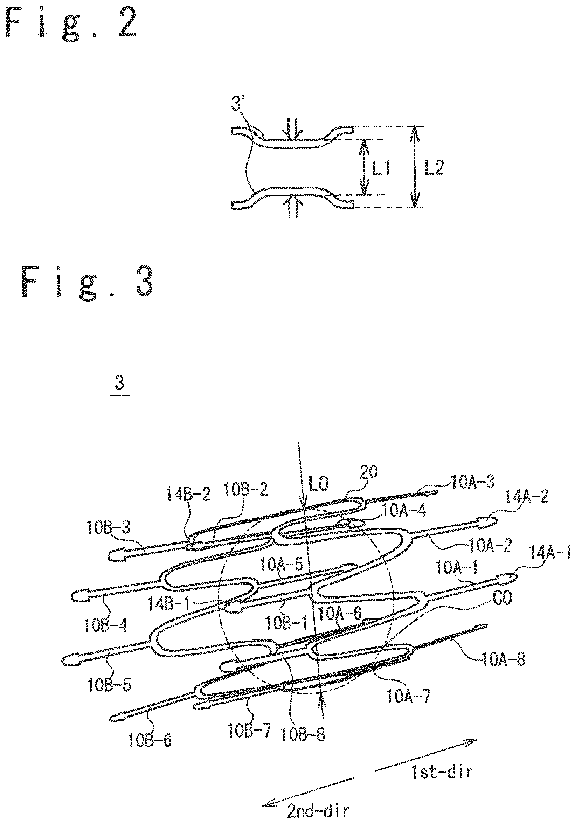

FIG. 2 is a diagram showing the contraction state of the anastomosis device. In the state that the central section of the anastomosis device 3' in the longitudinal direction is contracted by the holding tool (not shown), the outer diameter of the central section in the longitudinal direction is defined as L1 and an outer diameter of the end thereof is defined as being L2. When a value (L2/L1) when L2 is divided by L1 is a large value, it is difficult to insert the end of the anastomosis device 3' into the first tubular body or the second tubular body.

The anastomosis device 3 in an embodiment has such a structure that a value when L2 is divided by L1 is small. Therefore, it is easy to insert the end of the anastomosis device 3 into the first tubular body or the second tubular body.

### (Overview of anastomosis device)

FIG. 3 is a schematic perspective view of the anastomosis device 3 in the embodiment. The anastomosis device 3 is, for example, a stent-like anastomosis device (in other words, a stent for inosculation). The anastomosis device 3 is a anastomosis device used to inosculate the first tubular body (e.g. a first blood vessel, a first ureter, a first fallopian tube and so on) and the second tubular body (e.g. a second blood vessel, a second ureter, a second fallopian tube and so on). Note that at least one of the first tubular body and the second tubular body may be an artificial tubular body. The anastomosis device 3 is, for example, an anastomosis device used to inosculate the end of the first tubular body and the end of the second tubular body.

The anastomosis device 3 includes one corrugated circumference section 20, a plurality of top-side slender members (10A-1 to 10A-8), and a plurality of bottom-side slender members (10B-1 to 10B-8). The corrugated circumference section 20 is a portion where a wave-shaped structure is arranged in an annular manner, and a plurality of top sections and a plurality of bottom sections are alternately arranged in the wave-shaped structure.

Each of the plurality of top-side slender members (10A-1 to 10A-8) is connected to one of the plurality of top sections, and extends to a first direction (1st-dir) which is an outward direction of the corrugated circumference section 20 from the one top section. For example, the first direction (1st-dir) is a direction parallel to the longitudinal direction (the longitudinal directional axis) of the anastomosis device.

Each of the plurality of bottom-side slender members (10B-1 to 10B-8) is connected to one of the plurality of bottom sections, and extends to a second direction (2nd-dir) which is an outward direction of the corrugated circumference section 20 from the one bottom section. For example, the second direction is a direction opposite to the first direction.

Note that in an example shown in FIG. 3, the top-side is on the side of the first direction and the bottom-side is on the side of the second direction.
In the example shown in FIG. 3, the "top section" is a convex shape section arranged in a part of the corrugated circumference section 20 in the first direction and the "bottom section" is a convex shape section arranged in a part of the corrugated circumference section 20 in the second direction.

The corrugated circumference section 20, the plurality of top-side slender members (10A-1 to 10A-8), and the plurality of bottom-side slender members (10B-1 to 10B-8) are formed as a unitary body and configure one member.

### (Top-side slender member)

For example, the top-side slender member is configured from a linear member. Note that a linear member contains a wire-like member, a band-like member and so on. The total number of top-side slender members (10A-1 to 10A-8) is eight in the example shown in FIG. 3. However, the number of top-side slender members is not limited to the above-mentioned example and is optional. In the example shown in FIG. 3, the plurality of top-side slender members are respectively arranged in correspondence to the plurality of top sections of the corrugated circumference section 20. However, there may be a top section where the top-side slender member is not arranged. Also, in the example shown in FIG. 3, the plurality of top-side slender members are parallel to each other. For example, each top-side slender member is a straight-line like member, i.e. a top-side straight-line member.

Each top-side slender member 10A-N (when the total number of top-side slender members is M, N is a natural number in a range of 1 to M) has a first proximal end connected to a corresponding top section, and a first free end 14A-N. The first free end 14A-N is engageable with the first tubular body which is a biological tissue.

### (Bottom-side slender member)

For example, the bottom-side slender member is formed from the linear member. Note that a wire-like member, a band-like member and so on are contained in the linear member. The total number of the plurality of bottom-side slender members (10B-1 to 10B-8) is eight in the example shown in FIG. 3. However, the number of the bottom-side slender members is not limited to the above-mentioned example and is optional.

In the example shown in FIG. 3, the plurality of bottom-side slender members are respectively arranged in correspondence to the plurality of bottom sections of the corrugated circumference section 20. However, there may be a bottom section where any bottom-side slender member is not arranged. Also, in the example shown in FIG. 3, the plurality of bottom-side slender members are parallel to each other. For example, each bottom-side slender member is a straight-line like member, i.e. a bottom-side straight-line member.

Each bottom-side slender member 10B-N (when the total number of bottom-side slender members is M', N is a natural number in a range of 1 to M') has a second proximal end connected to a corresponding bottom section and a second free end 14B-N. The second free end 14B-N is engageable with the second tubular body which is a biological tissue.

### (Corrugated circumference section)

For example, the corrugated circumference section 20 is configured from the linear member. Note that a wire-like member, a band-like member and so on are contained in the linear member. The corrugated circumference section 20 is expandable along a circumference C0 (in other words, the corrugated circumference section 20 is expandable in a diameter direction), and is contractable along circumference C0 (in other words, the corrugated circumference section 20 is contractable in the diameter direction). That is, it could be said that the corrugated circumference section 20 is an expandable section. FIG. 3 shows the anastomosis device 3 in the expansion state. In the condition shown in FIG. 3, contraction force (that is, the force that makes the anastomosis device 3 contract in the diameter direction) is not given. When the contraction force does not act on the anastomosis device 3, the anastomosis device 3 maintains the expansion state. In the expansion state, the outer diameter of the central section of the anastomosis device in the longitudinal direction is an outer diameter L0. The outer diameter L0 is determined in consideration of the inner diameter of the tubular body which is an inosculation object. The outer diameter L0 is set to a value which is larger than the inner diameter of the tubular body as the inosculation object. For example, the outer diameter L0 is a value in the range of 1 mm to 5 mm.

### (Contraction state of anastomosis device)

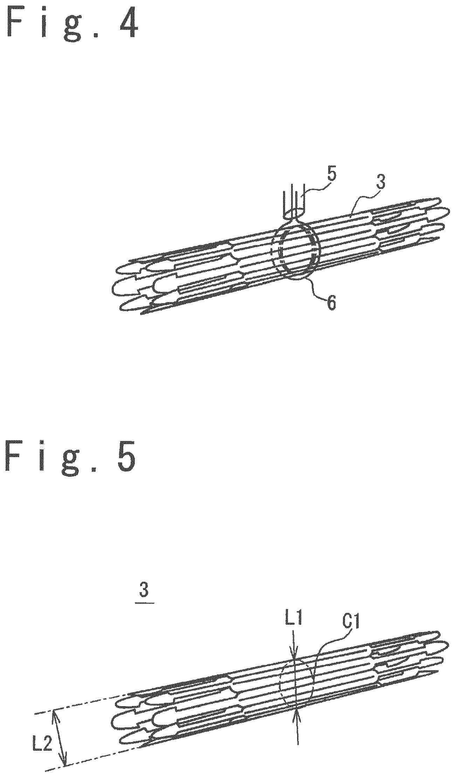

The anastomosis device 3 in the contraction state is shown in FIG. 4. The central section of the anastomosis device 3 in the longitudinal direction is held by a holding tool 5. A loop-like wire 6 extends from the distal end of the holding tool 5. By making the diameter of the loop like wire small, the anastomosis device 3 can be contracted. Note that a kind of the holding tool to contract the anastomosis device 3 is not limited to the example shown in FIG. 4.

The maximum contraction state of the anastomosis device 3 is shown in FIG. 5. The maximum contraction state is in a condition when the corrugated circumference section is most contracted. The corrugated circumference section is in an annular manner along the circumference C1. The outer diameter L1 of the central section of the anastomosis device 3 in the longitudinal direction in the maximum contraction state is determined in consideration of the inner diameter of the tubular body as the inosculation object. The outer diameter L1 is set to a value smaller than the inner diameter of the tubular body as the inosculation object. For example, the outer diameter L1 is a value in a range of 1 mm to 5 mm.

In the anastomosis device 3 of the embodiment, the expandable section of the anastomosis device 3 is configured from the corrugated circumference section 20. The stress and the warp are difficult to occur in a part of the corrugated circumference section 20 other than the top section and the bottom section. Therefore, when the contraction force acts on the central section of the anastomosis device 3 in the longitudinal direction, a difference between the outer diameter L1 in the central section of the corrugated circumference section 20 in the longitudinal direction and the outer diameter in the end of the corrugated circumference section 20 (in other words, the outer diameter in a cross section passing through the plurality of top sections of the corrugated circumference section 20, or, the outer diameter in the cross section passing through the plurality of bottom sections of the corrugated circumference section 20) can be made small. As a result, it is possible to make a value (L2/L1) small which is obtained by dividing the outer diameter L2 (the larger one of the outer diameter of the first end of the anastomosis device 3 on the first direction side and the outer diameter of the second end of the anastomosis device 3 on the second direction side) of the end section of the anastomosis device 3 by the outer diameter L1 of the central section in the longitudinal direction in the maximum contraction state. By making L2/L1 small, it becomes easy to insert one end of the anastomosis device 3 into the first tubular body or the second tubular body. In some embodiments, L2/L1 is set to be a value in a range of 1.0 to 1.3.

### (Detailed description of anastomosis device)

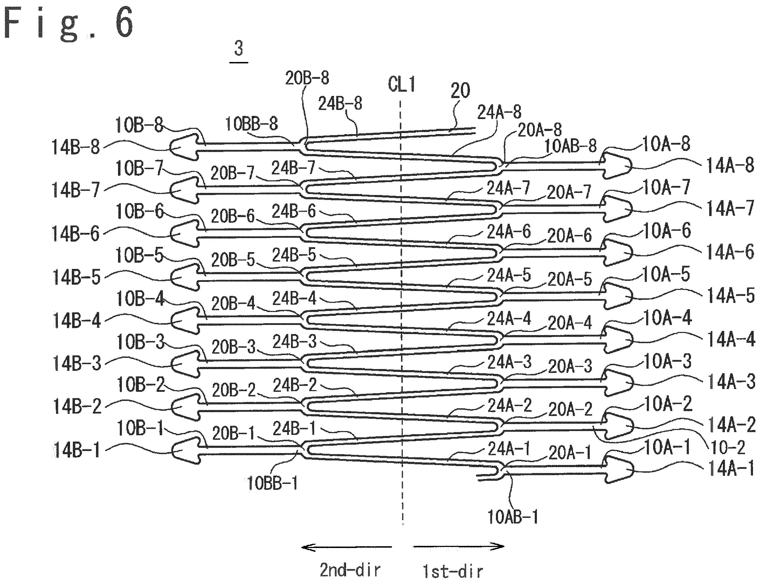

FIG. 6 is a development view of the anastomosis device 3 in the embodiment. For example, the anastomosis device 3 is a stent like anastomosis device (in other words, a stent for inosculation).

The anastomosis device 3 has only one corrugated circumference section 20, the plurality of top-side slender members (10A-1 to 10A-8), and the plurality of bottom-side slender members (10B-1 to 10B-8). Note that the number of the plurality of top-side slender members and the number of the plurality of bottom-side slender members are not limited to the above-mentioned example and are optional.

### (Corrugated circumference section)

The corrugated circumference section 20 has the plurality of top sections (20A-1 to 20A-8), the plurality of bottom sections (20B-1 to 20B-8), and a plurality of connection sections (24A-1 to 24A-8, 24B-1 to 24B-8) that connect between the plurality of top sections and the plurality of bottom sections. Each connection section may be configured from a straight-line like member (the straight-line member), or may be configured from a curve-like member (a curved member). For example, the corrugated circumference section 20 has a shape in which the plurality of top sections and the plurality of bottom section are alternately arranged, each top section is connected to two neighbor bottom sections by the connection sections, and each bottom section is connected to two neighbor top sections by the connection sections.

For example, the corrugated circumference section 20 is configured by a single corrugation. In other words, the corrugated circumference section 20 is not connected directly or indirectly to the other corrugated circumference section. When the corrugated circumference section 20 is connected directly or indirectly to the other corrugated circumference section, it is difficult that the corrugated circumference section 20 in the contraction of the anastomosis device 3 has a constant shape. As a result, there is a fear that L2/L1 in FIG. 5 becomes large. For example, the corrugated circumference section 20 has a unicursal shape with no end.

The first top section 20A-1 and the first bottom 20B-1 are connected by a first outbound-side connection section 24A-1. The first bottom section 20B-1 and the second top section 20A-2 are connected by a first inbound-side connection section 24B-1. In the same way, the N^{th} top section 20A-N (N is a natural number in a range of 1 to 7) and the N^{th} bottom section are connected by the N^{th} outbound-side connection section 24A-N. The N^{th} bottom section 20B-N (N is a natural number in a range of 1 to 7) and the (N+1)^{th} top section 20A-(N+1) are connected by the N^{th} inbound-side connection section 24B-N.

The eighth top section 20A-8 and the eighth bottom section 20B-8 are connected by an eighth outbound-side connection section 24A-8. The eighth bottom section 20B-8 and the first top section 20A-1 are connected by an eighth inbound-side connection section 24B-8. For example, the length of the plurality of outbound-side connection sections is possible to be made equal to each other. For example, the length of the plurality of inbound-side connection sections is possible to be made equal to each other. The length of each outbound-side connection section and the length of each inbound-side connection section are possible to be made equal to each other.

### (Top-side slender member)

The proximal end 10AB-1 of the first top-side slender member 10A-1 is connected to the first top section 20A-1. The first top-side slender member 10A-1 extends to the first direction (1st-dir) which is an outward direction of the corrugated circumference section 20 from the first top section 20A-1. The first top-side slender member 10A-1 has the first free end 14A-1. For example, the first free end 14A-1 has a first retaining section which is engaged with the first tubular body.

The proximal end 10AB-N (N is a natural number in a range of 1 to 8) of the N^{th} top-side slender member 10A-N is connected to the N^{th} top section 20A-N. The N^{th} top-side slender member 10A-N extends to the first direction (1st-dir) which is the outward direction of the corrugated circumference section 20 from the N^{th} top section 20A-N. The N^{th} top-side slender member 10A-N has the first free end 14A-N. For example, the first free end 14A-N has the first retaining section which is engaged with the first tubular body.

The length of the plurality of top-side slender members may be equal to each other. Alternatively, the length of the plurality of top-side slender members may be different from each other.

For example, each first retaining section has an arrowhead shape (approximate triangle shape) in which an angled section is rounded. For example, each first retaining section is configured by a plate-like member. In each first retaining section, a drawing resistance in a drawing-out direction from the first tubular body is larger than an insertion resistance in an insertion direction into the first tubular body. Note that the shape of the first retaining section is not limited to an arrowhead shape. The first retaining section may have the shape like a fold-back section of a fishing needle (in other words, the first retaining section may have a fold-back section).

### (Bottom-side slender member)

The proximal end 10BB-1 of the first bottom-side slender member 10B-1 is connected to the first bottom section 20B-1. The first bottom-side slender member 10B-1 extends to the second direction (2nd-dir) which is the outward direction of the corrugated circumference section 20 from the first bottom section 20B-1. The first bottom-side slender member 10B-1 has a second free end 14B-1. The second free end 14B-1 is a second retaining section which is engaged with the second tubular body.

The proximal end 10BB-N (N is a natural number in a range of 1 to 8) of the N^{th} bottom-side slender member 10B-N is connected to the N^{th} bottom section 20B-N. The N^{th} bottom-side slender member 10B-N extends to the second direction (2nd-dir) which is the outward direction of the corrugated circumference section 20 from the N^{th} bottom section 20B-N. The N^{th} bottom-side slender member 10B-N has the second free end 14B-N. For example, the second free end 14B-N has the second retaining section which is engaged with the second tubular body.

The length of the plurality of bottom-side slender members may be equal to each other. Alternatively, the length of the plurality of bottom-side slender member may be different from each other.

Each second retaining section has the arrowhead shape (approximate triangle shape) in which the angled section is rounded. For example, each second retaining section is configured from a plate-like member. In each second retaining section, a drawing-out resistance in a drawing-out direction from the second tubular body is larger than an insertion resistance in an insertion direction in the second tubular body. Note that the shape of the second retaining section is not limited to the arrowhead shape. The second retaining section may have a shape like a fold-back section of a fishing needle (in other words, the second retaining section may be composed of the fold-back section).

### (Expansion and contraction of anastomosis device)

By expanding the corrugated circumference section 20 along the direction of the circumference of the anastomosis device 3, the interval between the plurality of top sections expands. As a result, the interval between adjacent two of the plurality of top-side slender members expands. In expanding the corrugated circumference section 20 along the direction of circumference of the anastomosis device 3, the interval between adjacent two of the plurality of bottom sections expands. As a result, the interval between adjacent two of the plurality of bottom-side slender members expands. On the other hand, by contracting the corrugated circumference section 20 along the direction of circumference of the anastomosis device 3, the interval between adjacent two of the plurality of top sections reduces. As a result, the interval between adjacent two of the plurality of top-side slender members reduces. By contracting the corrugated circumference section 20 along the direction of circumference of the anastomosis device 3, the interval between adjacent two of the plurality of bottom sections reduces. As a result, the interval between adjacent two of the plurality of bottom-side slender members reduces.

### (Size of anastomosis device)

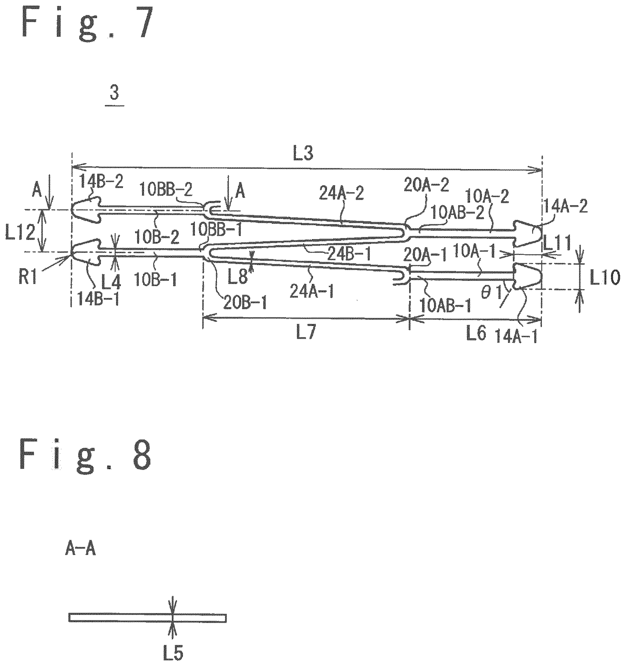

FIG. 7 is a development view showing a part of the anastomosis device 3 in a condition that the contraction force by the holding tool does not act. FIG. 8 is a sectional view viewed from the A-A plane in FIG. 7. The full length L3 of the anastomosis device 3 (along the longitudinal direction of the anastomosis device) is, for example, from 3 mm to 10 mm. When the full length L3 is less than 3 mm, there is a fear that the engaging of the anastomosis device 3 with the first tubular body or the second tubular body becomes insufficient. When the full length L3 is longer than 10 mm, there is a fear that the flexibility of the tubular body is lost in a joint section to the first tubular body or the second tubular body.

The width L4 (along the circumference direction of the anastomosis device 3) of each slender member (each top-side slender member or each bottom-side slender member) is, for example, from 0.05 mm to 0.2 mm. When the width L4 is less than 0.05 mm, there is a fear that the engaging of the anastomosis device 3 with the first tubular body or the second tubular body becomes insufficient. When the width L4 is wider than 0.2 mm, there is a fear that the flexibility of the tubular body is lost in the joint section to the first tubular body or the second tubular body.

The thickness L5 (along the radius direction of the anastomosis device 3) of each slender member (each top-side slender member or each bottom-side slender member) is, for example, from 0.03 mm to 0.15 mm. When the thickness L5 is less than 0.03 mm, there is a fear that the engaging of the anastomosis device 3 with the first tubular body or the second tubular body becomes insufficient. When the thickness L5 is thicker than 0.15 mm, there is a fear that the flexibility of the tubular body is lost in the joint section to the first tubular body or the second tubular body.

The length L6 of each slender member (each top-side slender member or each bottom-side slender member) is from 0.5 mm to 3 mm. When the length L6 is less than 0.5 mm, there is a fear that the engaging of the anastomosis device 3 with the first tubular body or the second tubular body becomes insufficient. When the length L6 is longer than 3 mm, there is a fear that the flexibility of the tubular body is lost in the joint section of the first tubular body or the second tubular body.

The length L7 of the corrugated circumference section 20 (along the longitudinal direction of the anastomosis device) in the expansion state is, for example, from 2 mm to 8 mm. When the length L7 is less than 2 mm, the expansion diameter is lacking, so that there is a fear that the engaging of the anastomosis device 3 with the first tubular body or the second tubular body becomes insufficient. When the length L7 is longer than 8 mm, there is a fear that the flexibility of the tubular body is lost in the joint section of the first tubular body or the second tubular body.

The width L8 of the corrugated circumference section 20 (in other words, the width of the linear member which configures the corrugated circumference section 20) is, for example, from 0.04 mm to 0.15 mm. When the width L8 is less than 0.04 mm, the expansion force is lacking, so that there is a fear that the engaging of the anastomosis device 3 to the first tubular body or the second tubular body becomes insufficient. When the width L8 is wider than 0.15 mm, there is a fear that the expansion force becomes excessive. Note that the width L8 of the corrugated circumference section 20 may be equal to the width L4 of each slender member. Alternatively, by making the width L8 of the corrugated circumference section 20 wider than the width L4 of each slender member, the engaging force of the anastomosis device 3 with the first tubular body or the second tubular body may be made large. Note that in order to enhance the diameter expansion force of the corrugated circumference section 20, the width L8 of the top-side or the bottom-side may be set to be larger than the width L8 in the central surface CL1.

The thickness of the corrugated circumference section 20 is, for example, from 0.03 mm to 0.15 mm. When the thickness is less than 0.03 mm, the expansion force is lacking, so that there is a fear that the engaging of the anastomosis device 3 to the first tubular body or the second tubular body becomes insufficient. When the thickness is 0.15 mm or above, there is a fear that the expansion force becomes excessive. Note that the thickness of the corrugated circumference section may be equal to the thickness L5 of each slender member. Alternatively, the thickness of the corrugated circumference section 20 may be made larger than the thickness L5 of each slender member, so that the engaging force with the first tubular body or the second tubular body is made larger. Or, the cross section of the corrugated circumference section (in other words, the cross section of the linear member which configures the corrugated circumference section 20) may be made larger than the cross section of each slender member, so that the engaging force to the first tubular body or the second tubular body is made large.

The width L10 of each retaining section (along the circumference direction of the anastomosis device 3) is, for example, from 0.1 mm to 0.3 mm. When the width L10 is less than 0.1 mm, there is a fear that the engaging of the anastomosis device 3 with the first tubular body or the second tubular body becomes insufficient. When the width L10 is wider than 0.3 mm, there is a fear that the engaging of the anastomosis device 3 with the first tubular body cr the second tubular body becomes excessive.

The length L11 of each retaining section (along the longitudinal direction of the anastomosis device 3) is, for example, from 0.15 mm to 0.6 mm. When the area of the retaining section is too large, there is a fear than the pushing force per unit area when the retaining section pushes the tubular body is lacking, so that the anastomosis device 3 slips down from the tubular body. Also, when the area of the retaining section is too small, there is a fear that the retaining section gets not to function as a portion that engages with the tubular body.

The angle θ1 between the rear end surface of each retaining section and the central axis of each slender member in the longitudinal direction is, for example, from 60 degrees to 90 degrees. When the angle θ1 is in a range from 60 degrees to 90 degrees, the retaining force of each retaining section becomes best.

The radius of curvature R1 of the rounded corner part at the tip section of each retaining section is from 0.03 mm to 0.1 mm. When the radius R1 is less than 0.03 mm, there is a fear that the penetration force to the biological tissue becomes excessive. When the radius R1 is over 0.1 mm, there is a fear that the width of the tip section of the retaining section is excessively large.

The interval L12 between adjacent two of the plurality of slender members (the interval between the top-side slender members in the expansion state or the interval between the bottom-side slender members in the expansion state) is from 0.8 mm to 2 mm. When the interval L12 is less than 0.8 mm, the expansion force is lacking, so that there is a fear that the engaging of the anastomosis device 3 with the first tubular body or the second tubular body becomes insufficient. When the interval L12 is over 2 mm, there is a fear that the expansion force becomes excessive.

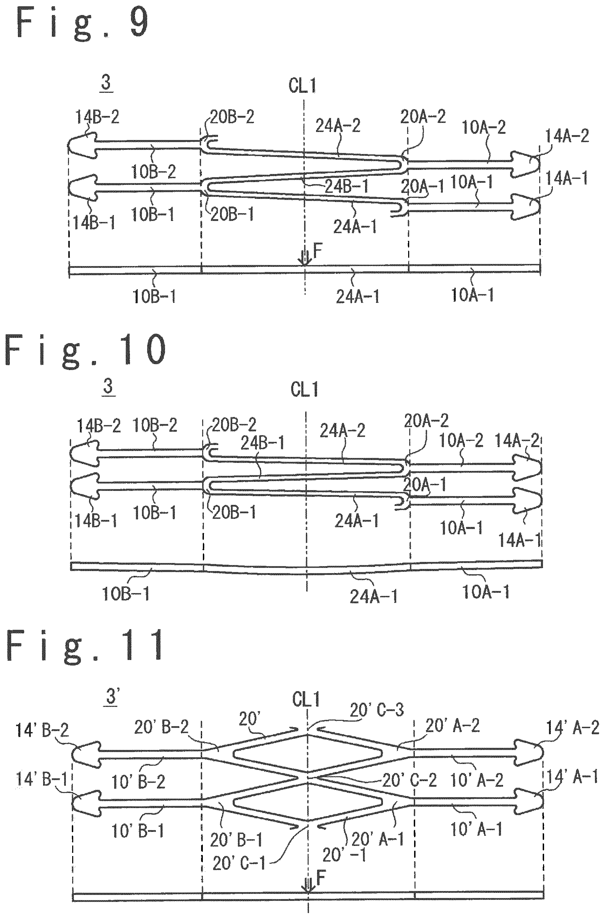

FIG. 9 to FIG. 12 is a simple diagram to show an effect of the corrugated circumference section.

In FIG. 9, it is supposed that the contraction force F acts on the central surface CL1 of the anastomosis device in the longitudinal direction. FIG. 9 is a diagram showing the condition before the contraction force F acts. FIG. 10 is a diagram showing the condition after the contraction force F acts. A part of the development view of the anastomosis device 3 in the embodiment is shown in the upper-side of FIG. 9. Also, the side view of the development view is shown in the lower-side of FIG. 9. In the same way, a part of the development view of the anastomosis device 3 in the embodiment is shown in the upper-side of FIG. 10. Also, the side view of the development view is shown in the lower-side of FIG. 10.

In an example shown in FIG. 9 and FIG. 10, the contraction force F acts directly on the central section of the anastomosis device 3 in the longitudinal direction. Due to the structural characteristic that there are few restraint parts in the corrugated circumference section 20, the stress and the warp are difficult to be caused in the part other than the top section and the bottom section. Therefore, when the contraction force F acts on the central section of the anastomosis device 3 in the longitudinal direction, a difference between the contraction degrees in the central section of the anastomosis device 3 in the longitudinal direction (in other words, the outer diameter in the central section in the longitudinal direction) and the contraction degrees in the end of the corrugated circumference section 20 (in other words, the outer diameter in the cross section which passes through the plurality of top sections or the outer diameter in the cross section which passes through the plurality of bottom sections) is small relatively. As a result, the difference between the contraction degrees on the central section of the anastomosis device 3 in the longitudinal direction (in other words, the outer diameter of the central section in the longitudinal direction) and the contraction degrees in the first free end 14A-N of the top-side slender member (in other words, the outer diameter in the first end of the anastomosis device 3 in the first direction) can be made small relatively. In the same way, the difference between the contraction degrees in the central section of the anastomosis device 3 in the longitudinal direction (in other words, the outer diameter of the central section in the longitudinal direction) and the contraction degrees in the second free end 14B-N of the bottom-side slender member (in other words, the outer diameter of the second end of the anastomosis device 3 in the second direction) can be made small relatively.

As described above, in an example shown in FIG. 9 and FIG. 10, L2/L1 (referring to FIG. 5) can be made small relatively. As a result, it becomes easy to insert the end of the anastomosis device 3 into the first tubular body or the second tubular body.

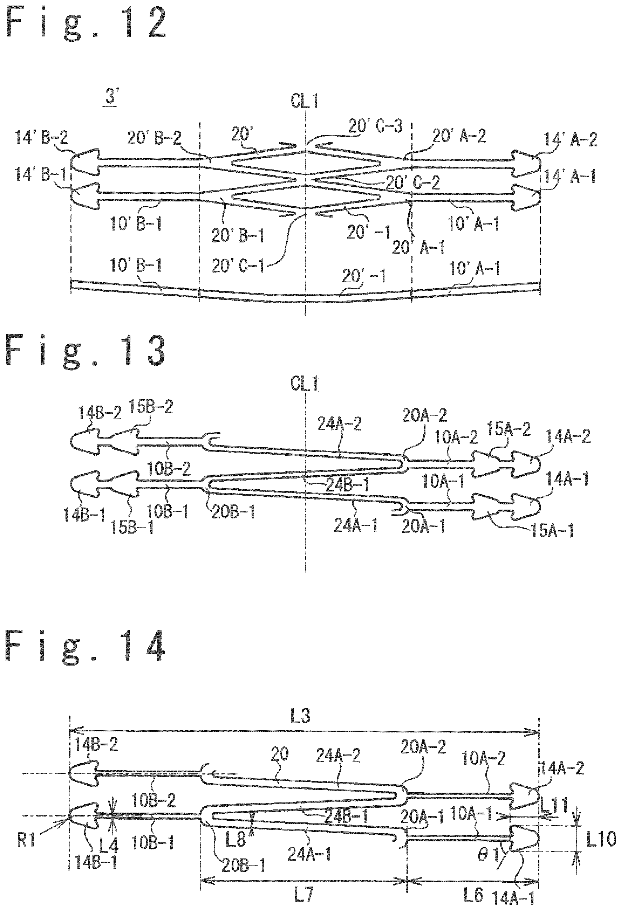

As a comparison example, the anastomosis device 3' having an expandable section 20' is shown in FIG. 11 and FIG. 12. The expandable section 20' is configured by a plurality of diamond-shaped members which are connected to each other along the circumference direction of the anastomosis device. The slender member 10'A-N and the slender member 10'B-N are connected respectively to the both ends of each diamond-shaped member. In FIG. 11, it is assumed that the contraction force F acts on the central surface of the anastomosis device 3' in the longitudinal direction CL1. FIG. 11 is a diagram showing the condition before the contraction force F acts. FIG. 12 is a diagram showing the condition after the contraction force F acts. A part of the development view of the anastomosis device 3' in the comparison example is shown in the upper-side of FIG. 11. Also, the side view of the development view is shown in the lower-side of FIG. 11. In the same way, the part of the development view of the anastomosis device 3' as the comparison example is shown in the upper-side of FIG. 12. Also, the side view of the development view is shown in the lower-side of FIG. 12.

In the example shown in FIG. 11 and FIG. 12, the contraction force F acts directly on the central section of the anastomosis device 3' in the longitudinal direction. A first diamond member 20'-1 which configures the expandable section 20' has four connection sections of the connection section 20'A-1, the connection section 20'B-1, the connection section 20'C-1, and the connection section 20'C-2. Therefore, the first diamond member 20'-1 is easy to generate the stress and the warp in a part except for the connection section 20'A-1 and the connection section 20'B-1. As for another diamond member, too, in the same way, it is easy to generate the stress and the warp. As a result, when the contraction force F acts on the central section of the anastomosis device 3' in the longitudinal direction, a difference between contraction degrees on the central section of the anastomosis device 3 in the longitudinal direction (in other words, the outer diameter of the central section in the longitudinal direction) and contraction degrees in the end of expandable section 20' (in other words, the outer diameter in the cross section which passes through the plurality of connection sections 20'A-N or the outer diameter in the cross section which passes through the plurality of connection sections 20'B-N) is relatively large. Therefore, the difference between contraction degrees in the central section of the anastomosis device 3' in the longitudinal direction (in other words, the outer diameter of the central section in the longitudinal direction) and contraction degrees in the first free end 14'A-N of the slender member 10'A-N (in other words, the outer diameter of first free end 14'A-N) is easy to become large relatively. In the same way, the difference between contraction degrees in the central section of the anastomosis device 3' in the longitudinal direction (in other words, the outer diameter of the central section in the longitudinal direction) and contraction degrees in the second free end 14'B-N of the slender member 10'B-N (in other words, the outer diameter of the second free end 14'3-N) is easy to become large relatively.

As mentioned above, in the example shown in FIG. 11 to FIG. 12, L2/L1 is easy to become large relatively (referring to FIG. 5). As a result, there is a possibility that it becomes difficult to insert the end of the anastomosis device 3' into the first tubular body or the second tubular body.

### (First modification example)

In FIG. 13, a modification example of the first free end of the top-side slender member 10A-N and the second free end of the bottom-side slender member 10B-N is shown. In an example shown in FIG. 13, a plurality of retaining sections 14A-N and 15A-N are provided for the first free end of the top-side slender member 10A-N. The retaining section 14A-N is provided for the tip of first free end. The retaining section 15A-N is provided on the side near the proximal end from the tip of first free end. Also, in the example shown in FIG. 13, the plurality of retaining sections 14B-N and 15B-N are provided in the second free end of the bottom-side slender member 10B-N. The retaining section 14B-N is provided for the tip of second free end, and the retaining section 15B-N is provided on the side nearer the proximal end than the tip of second free end. Some of the first free ends of the plurality of top-side slender members shown in FIG. 6 may be substituted by the first free end shown in FIG. 13. Alternatively, in all of the first free ends of the plurality of top-side slender members shown in FIG. 6 may be substituted by the first free ends shown in FIG. 13. Alternatively or additionally, some of the second free ends of the plurality of bottom-side slender members shown in FIG. 6 may be substituted by the second free ends shown in FIG. 13. Alternatively, in all of the second free ends of the plurality of bottom-side slender members shown in FIG. 6 may be substituted by the second free ends shown in FIG. 13.

### (Second modification example)

A modification example of the corrugated circumference section 20 is shown in FIG. 14. In an example of FIG. 14, the width L8 of each of the outbound-side connection sections 24A-N and the inbound-side connection sections 24B-N, which configure the corrugated circumference section 20, is wider than the width L4 of the slender member (the width of top-side slender member 10A-N or the width of bottom-side slender member 10B-N). Therefore, the expansion force of the corrugated circumference section 20 is enhanced. As a result, the engaging force to the first tubular body 3 or the second tubular body of the anastomosis device becomes large.

### (Third modification example)

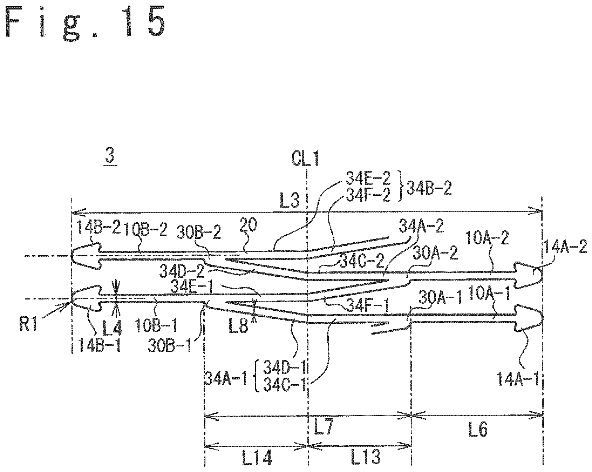

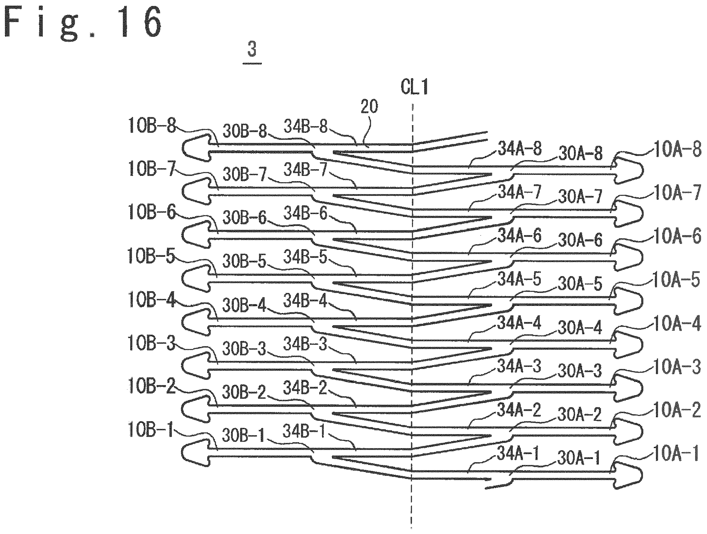

Another modification example of the corrugated circumference section 20 is shown in FIG. 15 and FIG. 16. FIG. 15 is a development view of a part of the anastomosis device 3 in the condition that the contraction force by the holding tool does not act. FIG. 16 is a development view of the anastomosis device 3 in the condition that the contraction force by the holding tool does not act on.

In an example shown in FIG. 15 and FIG. 16, the same reference numeral as used in FIG. 6 and FIG. 7 is assigned to a member having the same function as the member shown in FIG. 6 and FIG. 7. In the example shown in FIG. 15 and FIG. 16, the top section 20A-N, the bottom section 20B-N, the outbound-side connection section 24A-N, and the inbound-side connection section 24B-N in FIG. 6 and FIG. 7 are respectively substituted by a top section 30A-N, a bottom section 30B-N, an outbound-side connection section 34A-N, and an inbound-side connection section 34B-N. Also, in the example shown in FIG. 15 and FIG. 16, the top-side slender member 10A-N is a linear member, i.e. a top-side straight-line member. Moreover, in the example shown in FIG. 15 and FIG. 16, the bottom-side slender member 10B-N is a linear member, i.e. a bottom-side straight-line member.

Each outbound-side connection section 34A-N has a linear part, i.e. the outbound-side straight-line section 34C-N. The outbound-side straight-line section 34C-N extends from a corresponding top section 30A-N for the central surface CL1 of the anastomosis device 3 in the longitudinal direction. Each top-side slender member 10A-N and the corresponding outbound-side straight-line section 34C-N form one straight line. For example, to be understood from FIG. 15, the first top-side slender member 10A-1 and the first outbound-side straight-line section 34C-1 form one straight line. Also, the second top-side slender member 10A-2 and the second outbound-side straight-line section 34C-2 form one straight line. The N^{th} outbound-side straight-line section 34C-N and the corresponding bottom section 30B-N are connected by a part extending to the direction different from the longitudinal direction of the N^{th} top-side slender member 10A-N, that is, they are connected by the outbound-side different direction section 34D-N. For example, the first outbound-side straight-line section 34C-1 and the first bottom section 30B-1 are connected by the first outbound-side different direction section 34D-1. Also, the second outbound-side straight-line section 34C-2 and the second bottom section 30B-2 are connected by the second outbound-side different direction section 34D-2.

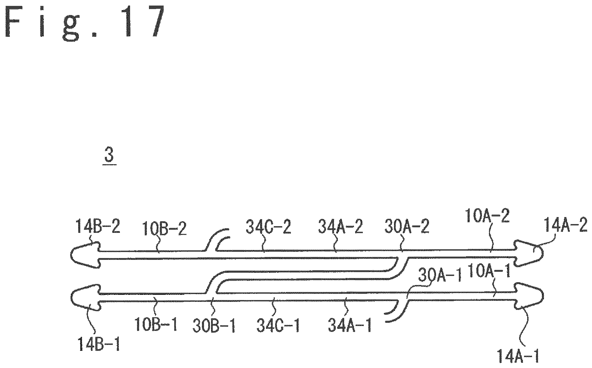

Note that as shown in FIG. 17, each outbound-side connection section 34A-N may not have a different direction part, i.e. the outbound-side different direction section 34D-N.

The value (L13/L7) when the length L13 of each outbound-side straight-line section 34C-N is divided by the length L7 of the corrugated circumference section 20 in the expansion state is, for example, 0.1 or above and 1 or below. The value (L13/L7) which the length L13 of each outbound-side straight-line section 34C-N is divided by the length L7 of the corrugated circumference section 20 in the expansion state is desirable to be 0.5 or above and 1 or below. This is because in case of L13/L7 equal to or more than 0.5, one straight line configured by the top-side slender member 10A-N and the outbound-side straight-line section 34C-N crosses on the central surface CL1 in the longitudinal direction. As a result, the contraction force which acts on the central section of the anastomosis device 3 in the longitudinal direction acts directly on the one straight line, too, the reduction of the outer diameter of the first end of the anastomosis device 3 (the end on the side of first free end 14A-N) becomes surer. Note that in an example shown in FIG. 15, the value (L13/L7) when the length L13 of each outbound-side straight-line section 34C-N is divided by the length L7 of the corrugated circumference section 20 in the expansion state is 0.5.

Each inbound-side connection section 34B-N has a linear section, that is, has the inbound-side straight-line section 34E-N. The inbound-side straight-line section 34E-N extends from the corresponding bottom section 30B-N for the central surface CL1 of the anastomosis device 3 in the longitudinal direction. Each bottom-side slender member 10B-N and the corresponding inbound-side straight-line section 34E-N form one straight line. For example, as understood from FIG. 15, the first bottom-side slender member 10B-1 and the first inbound-side straight-line section 34E-1 form one straight line. Also, the second bottom-side slender member 10B-2 and the second-side straight-line section 34E-2 form one straight line. The N^{th} inbound-side straight-line section 34E-N and the corresponding top section 30A-N+1 is connected by a part extending to a direction different from the longitudinal direction of the N^{th} bottom-side slender member 10B-N, that is, the inbound-side different direction section 34F-N. For example, the first inbound-side straight-line section 34E-1 and the second top section 30A-2 are connected by the first inbound-side different direction section 34F-1. Also, the second-side straight-line section 34E-2 and the third top section 30A-3 are connected by the second-side different direction section 34F-2.

The value (L14/L7) when the length L14 of each inbound-side straight-line section 34E-N is divided by the length L7 of the corrugated circumference section 20 in the expansion state is, for example, 0.1 or above and 1 or below. The value (L14/L7) when the length L14 of each inbound-side straight-line section 34E-N is divided by the length L7 of the corrugated circumference section 20 in the expansion state is desirably 0.5 or more and 1 or below. This is because by making L14/L7 equal to or more than 0.5, one straight line configured by the bottom-side slender member 10B-N and the inbound-side straight-line section 34E-N crosses the central surface CL1 in the longitudinal direction. As a result, the contraction force which acts on the central section of the anastomosis device 3 in the longitudinal direction acts directly on the one straight line, too. The reduction of the outer diameter in the second end of the anastomosis device 3 (the end on the side of second free end 14B-N) becomes surer. Note that in the example shown in FIG. 15, the value (L14/L7) when the length L14 of each inbound-side straight-line section 34E-N is divided by the length L7 of the corrugated circumference section 20 in the expansion state is 0.5.

### (Fourth modification example)

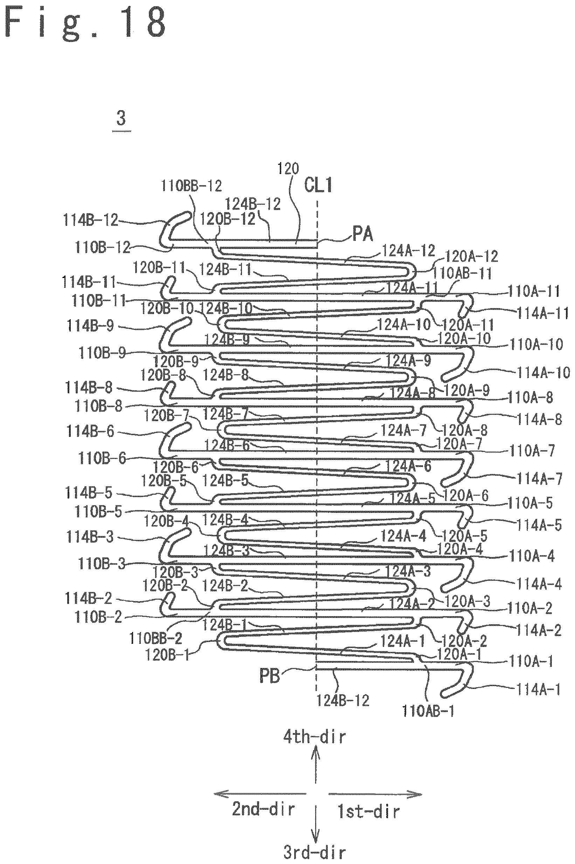

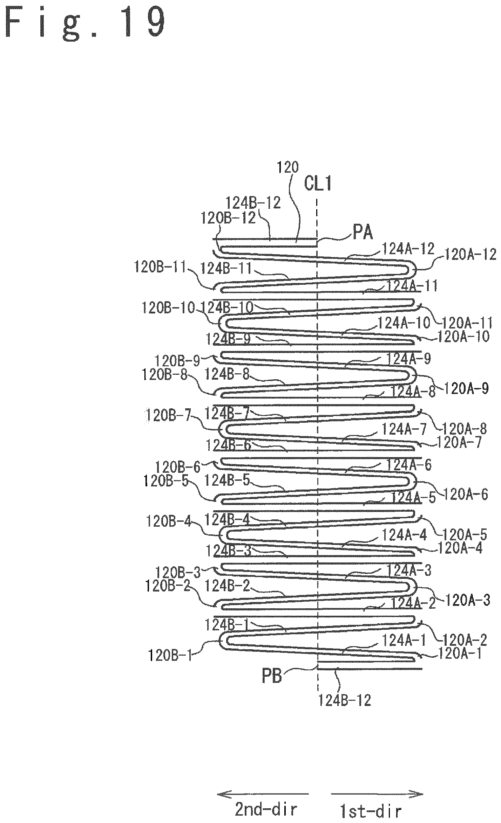

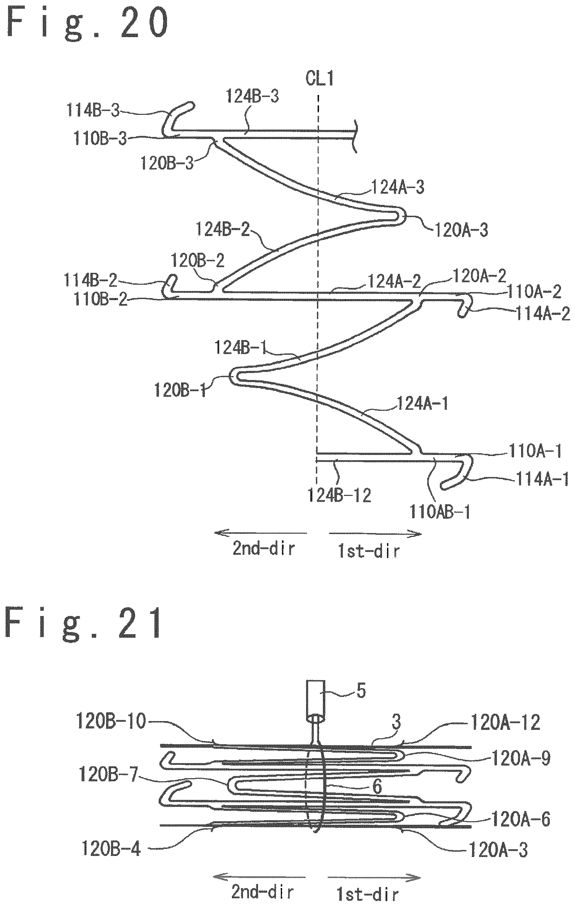

Another modification example of the corrugated circumference section and the retaining section is shown in FIG. 18 to FIG. 20. FIG. 18 is a development view of the anastomosis device 3 in the condition (the diameter contraction state) that the contraction force by the holding tool acts. FIG. 19 is a development view of the corrugated circumference section 120. FIG. 20 is a development view of a part of the anastomosis device 3 in the condition (the diameter expansion state) that the contraction force by the holding tool does not act.

The anastomosis device 3 has only one corrugated circumference section 120, a plurality of top-side slender members (110A-1, 110A-2, 110A-4, 110A-5, 110A-7, 110A-8, 110A-10, 110A-11), and a plurality of bottom-side slender members (110B-2, 110B-3, 110B-5, 110B-6, 110B-8, 110B-9, 110B-11, 110B-12). Note that the number of the plurality of top-side slender members and the number of the plurality of bottom-side slender members are optional.

### (Corrugated circumference section)

A part PA and a part PB of a corrugated circumference section 120 in FIG. 18 are actually connected, and the corrugated circumference section 120 has a circular shape as a whole. The corrugated circumference section 120 has a plurality of top sections (120A-1 to 120A-12), a plurality of bottom sections (120B-1 to 120B-12), and a plurality of connection sections (124A-1, to 124A-12, 124B-1 to 124B-12) which connects the plurality of top sections and the plurality of bottom sections. Each connection section may be configured by a straight-line like member (the straight-line member) or a curved member (a curve member). The corrugated circumference section 120 includes the plurality of top sections and the plurality of bottom sections arranged alternately, and has the shape in which each top section is connected to two neighbor bottom sections by the connection sections, and each bottom section is connected to the two neighbor top sections by the connection sections.

For example, the corrugated circumference section 120 is configured to have a single corrugation. In other words, the corrugated circumference section 120 is not connected directly or indirectly with another corrugated circumference section. When the corrugated circumference section 120 is connected directly or indirectly with the other corrugated circumference section, the corrugated circumference section 120 is difficult to take a constant shape in the contraction of the anastomosis device 3. As a result, there is a fear that L2/L1 (referring to FIG. 5) becomes large. For example, the corrugated circumference section 120 has the unicursal shape with no end. FIG. 19 shows a development view of the corrugated circumference section 120.

In the example shown in FIG. 18, the corrugated circumference section 120 includes the plurality of straight-line sections (124B-12; 124A-2; 124B-3; 124A-5; 124B-6; 124A-8; 124B-9; 124A-11) parallel to the longitudinal direction of the anastomosis device, the plurality of first protruding sections (124B-2, 120A-3, 124A-3; 124B-5, 120A-6, 124A-6; 124B-8, 120A-9, 124A-9; 124B-11, 120A-12, 124A-12) which protrude for the first direction side, the plurality of second protruding sections (124A-1, 120B-1, 124B-1; 124A-4, 120B-4, 124B-4; 124A-7, 120B-7, 124B-7; 124A-10, 120B-10, 124B-10) which protrude for the second direction side. The first protruding section as one of the plurality of first protruding sections is configured from the second-side connection section 124B-2, the third top section 120A-3, and the third outbound-side connection section 124A-3. The second protruding section as one of the plurality of second protruding sections is configured from, for example, the first outbound-side connection section 124A-1, the first bottom section 120B-1 and the first inbound-side connection section 124B-1. Each first protruding section oversteps the central surface CL of the anastomosis device in the longitudinal direction to protrude to the first direction side. Each second protruding section oversteps the central surface CL of the anastomosis device in the longitudinal direction to protrude to the second direction side. In the example shown in FIG. 18, each first protruding section is arranged between two straight-line parts (i.e., between the two straight-line sections connected with each first protruding section). Also, each second protruding section is arranged between two straight-line parts (i.e., between the two straight-line parts connected with each second protruding section). Also, the first protruding section and the second protruding section are alternately arranged along the circumferential direction of the anastomosis device which is the direction perpendicular to the first direction.

The first top section 120A-1 and the first bottom section 120B-1 are connected by the first outbound-side connection section 124A-1. The first bottom section 120B-1 and the second top section 120A-2 are connected by the first inbound-side connection section 124B-1. In the same way, the N^{th} top section 120A-N (N is any natural number in a range of 1 to 11) and the N^{th} bottom section are connected by the N^{th} outbound-side connection section 124A-N. The N^{th} bottom section 120B-N (N is any natural number in a range of 1 to 11) and the (N+1)^{th} top section 120A-(N+1) are connected by the N^{th} inbound-side connection section 124B-N. The twelfth top section 120A-12 and the twelfth bottom section 120B-12 are connected by the twelfth outbound-side connection section 124A-12. The twelfth bottom section 120B-12 and the first top section 120A-1 are connected by the twelfth inbound-side connection section 124B-12. In the example shown in FIG. 18, some outbound-side connection sections of the plurality of outbound-side connection sections are parallel to the longitudinal direction of the anastomosis device 3, but the remaining outbound-side connection sections of the plurality of outbound-side connection sections are not parallel to the longitudinal direction of the anastomosis device 3.
In the same way, in the example shown in FIG. 18, some inbound-side connection sections of the plurality of inbound-side connection sections are parallel to the longitudinal direction of the anastomosis device 3, but the remaining inbound-side connection sections of the plurality of inbound-side connection section are not parallel to the longitudinal direction of the anastomosis device 3.

Note that in the example shown in FIG. 18, the twelfth inbound-side connection section 124B-12 crosses the central surface CL1 of the anastomosis device 3 in the longitudinal direction. Also, in the example shown in FIG. 18, all of the outbound-side connection sections and all of the inbound-side connection sections cross on the central surface CL1 of the anastomosis device 3 in the longitudinal direction, respectively.

### (Top-side slender member)

A proximal end 110AB-1. of the first top-side slender member 110A-1 is connected to the first top section 120A-1. The first top-side slender member 110A-1 extends from the first top section 120A-1 toward the first direction (1st-dir), which is the outward direction of the corrugated circumference section 120. The first top-side slender member 110A-1 has a first free end 114A-1. For example, the first free end 114A-1 has a first retaining section which is engaged with the first tubular body. A part of the first top-side slender member 110A-1 (more specifically, a part except for the first free end 114A-1) and a part of the corrugated circumference section 120 (more specifically, the twelfth inbound-side connection section 124B-12) may be arranged to form a straight line or to form parallel lines.

A proximal end 110AB-P (only the proximal end 110AB-1 and a proximal end 110AB-11 are assigned with reference numerals in FIG. 18) of the P^{th} top-side slender member 110A-P (P is a natural number in a range of 1, 2, 4, 5, 7, 8, 10, and 11) is connected to the P^{th} top section 120A-P. The P^{th} top-side slender member 110A-P extends from the P^{th} top section 120A-P for the first direction (1st-dir) which is the outward direction of the corrugated circumference section 120. The P^{th} top-side slender member 110A-P has a first free end 114A-P. For example, the first free end 114A-P has a first retaining section which is engaged with the first tubular body. A part of the P^{th} top-side slender member 110A-P and a part of the corrugated circumference section 120 (more specifically, the P^{th} inbound-side connection section which is connected to the P^{th} top section or the P^{th} outbound-side connection section 124A-P which is connected to the P^{th} top section) may be arranged in a straight line or in parallel.

The plurality of top-side slender members may be equal to each other in length. Alternatively, the plurality of top-side slender members may be different from each other in length. Note that in the example shown in FIG. 18, any top-side slender member is not provided for the third top section 120A-3, the sixth top section 120A-6, the ninth top section 120A-9, and the twelfth top section 120A-12.

For example, each first retaining section has a fold-back section. The tip of the fold-back section is located on the second direction side (2nd-dir) from the fold-back section proximal end. Each fold-back section is provided on the third direction (3rd-dir) side to the top-side slender member (that is, to a corresponding top-side slender member) for which each fold-back section is provided. Note that the third direction is a direction perpendicular to the first direction. Alternatively, each fold-back section may be provided on the side of fourth direction (4th-dir) which is a direction opposite to the third direction, to the top-side slender member for which each fold-back section is provided (that is, to a corresponding top-side slender member). By setting the direction of each fold-back section to the direction of the corresponding top-side slender member, a risk that the neighbor fold-back sections are interwined with each other can be reduced.

### (Bottom-side slender member)

The proximal end 110BB-2 of the second bottom-side slender member 110B-2 is connected to the second bottom section 120B-2. The second bottom-side slender member 110B-2 extends from the second bottom section 120B-2 for the second direction (2nd-dir) which is the outward direction of the corrugated circumference section 20. The second bottom-side slender member 110B-2 has a second free end 114B-2. For example, the second free end 114B-2 has a second retaining section that is engaged with the second tubular body. A part of the corrugated circumference section 120 (more specifically, the second outbound-side connection section 124A-2) and the second bottom-side slender member 110B-2 may be arranged on a straight line or they may be arranged in parallel.

A proximal end 110BB-Q of the Q bottom-side slender member 110B-Q (Q is a natural number in a range of 2, 3, 5, 6, 8, 9, 11, and 12) is connected to the Q bottom section 120B-Q. The Q bottom-side slender member 110B-Q extends from the Q^{th} bottom section 120B-Q for the second direction (2nd-dir) that is the outward direction of the corrugated circumference section 120. The Q^{th} bottom-side slender member 110B-Q has a second free end 114B-Q. For example, the second free end 114B-Q has a second retaining section that is engaged with the second tubular body. A part of the Q^{th} bottom-side slender member 110B-Q and a part of the corrugated circumference section 120 (more specifically, the outbound-side connection section connected to the Q^{th} bottom section or the Q^{th} inbound-side connection section 124B-Q connected to the Q^{th} bottom section) may be arranged in a straight line or may be arranged in parallel.

Note that in the example shown in FIG. 18, a part of the first top-side slender member 110A-1 (more specifically, a part except for the first free end 114A-1), a part of the corrugated circumference section 120 (more specifically, the twelfth inbound-side connection section 124B-12) and a part of the twelfth bottom-side slender member 110B-12 (more specifically, a part except for the second free end 114B-12) are arranged in a straight line.

The plurality of bottom-side slender members may be equal to each other in length. Alternatively, the plurality of bottom-side slender members may be different from each other in length. Note that in the example shown in FIG. 18, any bottom-side slender member is not provided for the first bottom section 120B-1, the fourth bottom section 120B-4, the seventh bottom section 120B-7, and the tenth bottom section 120B-10.

For example, each second retaining section has the fold-back section. The tip of the fold-back section is situated on the side of first direction (1st-dir) from the proximal end of the fold-back section. Each fold-back section is provided on the side of fourth direction (4th-dir) to the bottom-side slender member in which each concerned fold-back section is provided (that is, to a corresponding bottom-side slender member). Alternatively, each fold-back section may be provided on the side of third direction (3rd-dir) to the bottom-side slender member for which each fold-back section is provided (that is, to a corresponding bottom-side slender member). By making a direction to which each fold-back section is provided to an identical direction to the corresponding bottom-side slender member, a risk that the neighbor fold-back sections are connected with each other is reduced.

### (Extension and contraction of anastomosis device)

By the corrugated circumference section 120 expanding along the direction of the circumference of the anastomosis device 3, the distance between the plurality of top sections expands. As a result, the distance between the plurality of top-side slender members expands. By the corrugated circumference section 120 expanding along the direction of the circumference of the anastomosis device 3, the interval between the plurality of bottom sections expands. As a result, the interval between the plurality of bottom-side slender members expands. On the other hand, because the corrugated circumference section 120 contracts along the direction of the circumference of the anastomosis device 3, the interval between the plurality of top sections reduces. As a result, the interval between the plurality of top-side slender members reduces. By the corrugated circumference section 120 contracting along the direction of the circumference of the anastomosis device 3, the interval between the plurality of bottom sections reduces. As a result, the interval between the plurality of bottom-side slender members reduces. FIG. 20 is a development view of a part of the anastomosis device 3 in the state (the diameter expansion state) that the contraction force by the holding tool does not act.

Regarding each size of the anastomosis device 3 (each size in the diameter expansion state), the full length L3 of the anastomosis device 3 (the length of the anastomosis device along the longitudinal direction), the width L4 (the length of the anastomosis device 3 along the circumference direction) of each slender member (each top-side slender member or each bottom-side slender member), the thickness L5 (the length of the anastomosis device 3 along the direction of the radius) of each slender member (each top-side slender member or each bottom-side slender member), the length L6 of each slender member (each top-side slender member or each bottom-side slender member), the length L7 of the corrugated circumference section 120 in the expansion state (the length of the anastomosis device along the longitudinal direction), and the width L8 of the corrugated circumference section 20 may be equal to the sizes L3, L4, L5, L6, L7, and L8 which have been explained with reference to FIG. 7, respectively.

It would be apparent that the example shown in FIG. 18 to FIG. 20 attains the effect similar to the examples shown in FIG. 3 to FIG. 8.

In addition, in the example shown in FIG. 18 to FIG. 20, when the corrugated circumference section 120 has a first protruding section protruding to a first direction side (124B-2, 120A-3, 124A-3; 124B-5, 120A-6, 124A-6; 124B-8, 120A-9, 124A-9; 124B-11, 120A-12, 124A-12), and a second protruding section protruding to a second direction side (124A-1, 120B-1, 124B-1; 124A-4, 120B-4, 124B-4; 124A-7, 120B-7, 124B-7; 124A-10, 120B-10, 124B-10), the effect can be attained that the anastomosis device 3 is difficult to fall down from the holding tool. The effect will be described with reference to FIG. 21.

FIG. 21 is a schematic side view of the anastomosis device 3 in the condition that the contraction force by the holding tool acts (the diameter contraction state). By the loop-like wire 6 of the holding tool 5, when the neighborhood of the central surface of the anastomosis device 3 in the longitudinal direction is held, the third top section 120A-3, the sixth top section 120A-6, the ninth top section 120A-9, the twelfth top section 120A-12, the first bottom section 120B-1, the fourth bottom section 120B-4, the seventh bottom section 120B-7, the tenth bottom section 120B-10 are a part that the diameter is difficult to be contracted. The third top section 120A-3, the sixth top section 120A-6, the ninth top section 120A-9, the twelfth top section 120A-12, the first bottom section 120B-1, the fourth bottom section 120B-4, the seventh bottom section 120B-7, the tenth bottom section 120B-10 in which the diameter is difficult to be contracted, functions as a stopper to the loop-like wire 6 of the holding tool 5. That is, when the loop-like wire 6 moves along the longitudinal direction of the anastomosis device 3, the stopper limits the movement of the loop like wire 6. Therefore, the anastomosis device 3 is difficult to be fallen down from the holding tool 5.

The first protruding section can be said as a first spring part. In the example shown in FIG. 18, the corrugated circumference section 120 has four first spring parts. Each first spring part has a convex shape for the first direction side. Each first spring part crosses the central surface CL1 of the anastomosis device 3 in the longitudinal direction. The second protruding section can be said as a second spring part. In the example shown in FIG. 18, the corrugated circumference section 120 has four second spring parts. Each second spring part has a convex shape for the second direction side. Each second spring part crosses the central surface CL1 of the anastomosis device 3 in the longitudinal direction. Note that in the example shown in FIG. 18, the first spring part and the second spring part are alternately arranged along the circumferential direction of the anastomosis device 3.

### (Fifth modification example)

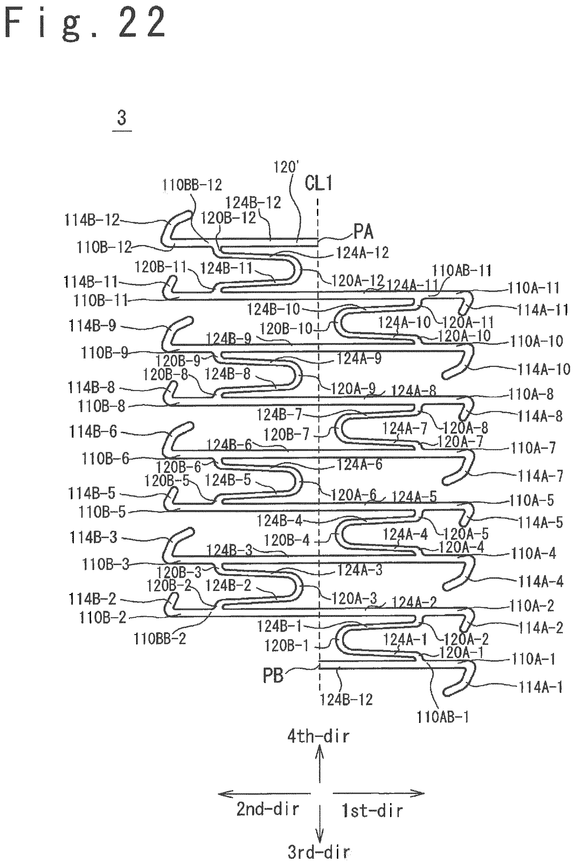

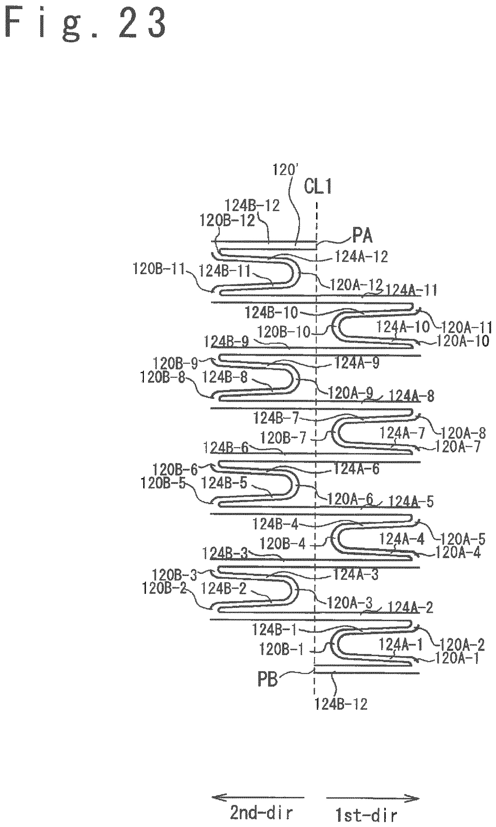

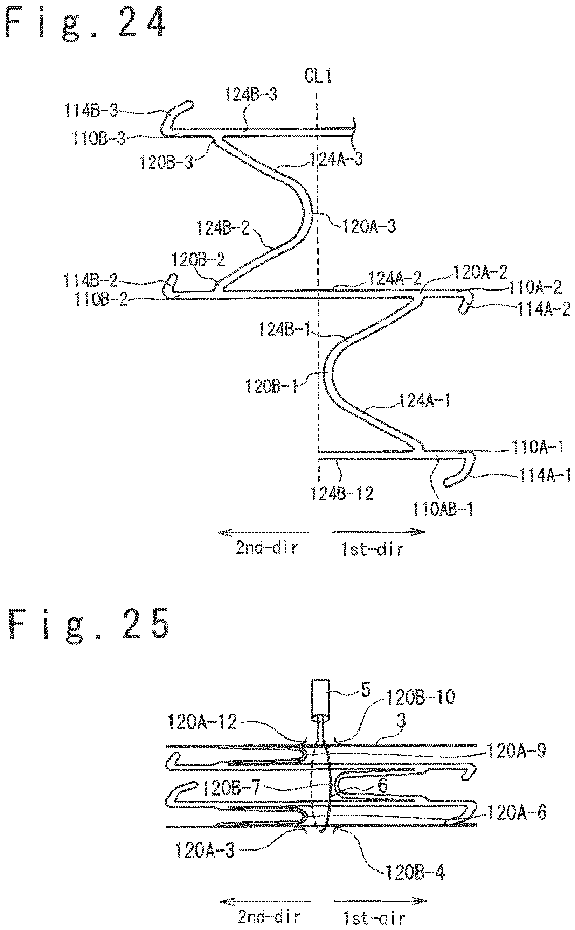

Another modification example of the corrugated circumference section is shown in FIG. 22 to FIG. 25. FIG. 22 is a development view of the anastomosis device 3 in the condition that the contraction force by the holding tool acts (the diameter contraction state). FIG. 23 is a development view of the corrugated circumference section 120'. FIG. 24 is a development view of a part of the anastomosis device 3 in the condition that the contraction force by the holding tool does not act (the diameter expansion state). FIG. 25 is a schematic side view of the anastomosis device 3 in the condition that the contraction force by the holding tool acts (the diameter contraction state).

In FIG. 22 to FIG. 25, the same reference numeral is assigned to a member having the same function as described with reference to FIG. 18 to FIG. 21. In an example shown in FIG. 22 to FIG. 25, the shape of corrugated circumference section 120' is different from the shape of corrugated circumference section 120 shown in FIG. 18 to FIG. 21. Regarding the corrugated circumference section 120 shown in FIG. 22 to FIG. 25, the lengths of the outbound-side connection section and the inbound-side connection section are shorter than the lengths of the outbound-side connection section and the inbound-side connection section shown in FIG. 18 to FIG. 21. The other points of the example shown in FIG. 22 to FIG. 25 are same as those of the example shown in FIG. 18 to FIG. 21.

In the example shown in FIG. 22 to FIG. 25, the corrugated circumference section 120' has straight-line parts (124B-12, 124A-2, 124B-3, 124A-5, 1243-6, 124A-8, 124B-9, 124A-11) parallel to the longitudinal direction of the anastomosis device, first protruding sections (124B-2, 120A-3, 124A-3; 124B-5, 120A-6, 124A-6; 124B-8, 120A-9, 124A-9; 124B-11, 120A-12, 124A-12) protruding for the side of first direction, and second protruding sections (124A-1, 120B-1, 124B-1; 124A-4, 120B-4, 124B-4; 124A-7, 120B-7, 124B-7; 124A-10, 120B-10, 124B-10) protruding for the side of second direction. The first protruding sections protruding for the side of first direction (e.g. the third top section 120A-3, the sixth top section 120A-6, the ninth top section 120A-9, and the twelfth top section 120A-12) do not reach the central surface CL of the anastomosis device in the longitudinal direction (in other words, the first protruding section do not intersect with the central surface CL of the anastomosis device in the longitudinal direction). The second protruding sections (e.g. the first bottom section 120B-1, the fourth bottom section 120B-4, the seventh bottom section 120B-7, and the tenth bottom section 120B-10) do not reach the central surface CL of the anastomosis device in the longitudinal direction (in other words, the second protruding section does not intersect with the central surface CL of the anastomosis device in the longitudinal direction). The first protruding section and the second protruding section are arranged along the longitudinal direction of the anastomosis device so as not to overlap each other. In other words, the first protruding section and the second protruding section are arranged so as not to overlap each other when viewed from a direction perpendicular to the longitudinal direction of the anastomosis device. In the example shown in FIG. 22 to FIG. 25, each first protruding section is arranged between two straight-line parts (that is, between the two straight-line parts connected with each first protruding section). Also, each second protruding section is arranged between the two straight-line parts (that is, between two straight-line parts connected with each second protruding section). Also, the first protruding sections and the second protruding sections are alternately arranged along the circumferential direction of the anastomosis device 3, which is a direction perpendicular to the first direction.

In the example shown in FIG. 22, each of the second outbound-side connection section 124A-2, the third inbound-side connection section 124B-3, the fifth outbound-side connection section 124A-5, the sixth inbound-side connection section 124B-6, the eighth outbound-side connection section 124A-8, the ninth inbound-side connection section 124B-9, the eleventh outbound-side connection section 124A-11, and the twelfth inbound-side connection section 124B-12 crosses the central surface CL1 of the anastomosis device 3 in the longitudinal direction. On the other hand, the outbound-side connection sections and the inbound-side connection sections except for them do not cross the central surface CL1 of the anastomosis device 3 in the longitudinal direction. In other words, each of the outbound-side connection section and the inbound-side connection section which are parallel to or on a line with the first top-side slender member 110A-1 crosses the central surface CL1 of the anastomosis device 3 in the longitudinal direction. On the other hand, each of the outbound-side connection section and the inbound-side connection section which are parallel to or on a line with the first top-side slender member 110A-1 does not cross the central surface CL1 of the anastomosis device 3 in the longitudinal direction.

It would be apparent that the example shown in FIG. 22 to FIG. 25 can attain the same effect as in the example shown in FIG. 3 to FIG. 8.

In addition, in the example shown in FIG. 22 to FIG. 25, when the corrugated circumference section 120' has the first protruding sections (124B-2, 120A-3, 124A-3; 124B-5, 120A-6, 124A-6; 124B-8, 120A-9, 124A-9; 124B-11, 120A-12, 124A-12) protruding for the side of first direction, and the second protruding sections (124A-1-, 120B-1, 124B-1; 124A-4, 120B-4, 124B-4; 124A-7, 120B-7, 124B-7; 124A-10, 120B-10, 124B-10) protruding for the side of second direction, the effect can be attained that the anastomosis device 3 is difficult to fall down from the holding tool. The effect will be described with reference to FIG. 25.

FIG. 25 is a schematic side view of the anastomosis device 3 in the condition that the contraction force by the holding tool acts (the diameter contraction state). When the neighborhood of the central surface of the anastomosis device 3 in the longitudinal direction is held by the loop-like wire 6 of the holding tool 5, the third top section 120A-3, the sixth top section 120A-6, the ninth top section 120A-9, the twelfth top section 120A-12, the first bottom section 120B-1, the fourth bottom section 120B-4, the seventh bottom section 120B-7, the tenth bottom section 120B-10 are parts that are difficult to be contracted in diameter. The third top section 120A-3, the sixth top section 120A-6, the ninth top section 120A-9, the twelfth top section 120A-12, the first bottom section 120B-1, the fourth bottom section 120B-4, the seventh bottom section 120B-7, the tenth bottom section 120B-10 which are difficult to be contracted in diameter, function as the stopper to the loop-like wire 6 of the holding tool 5. That is, when the loop-like wire 6 moves along the longitudinal direction of the anastomosis device 3, the stopper limits the movement of the loop-like wire 6. Therefore, the anastomosis device 3 is difficult to fall down from the holding tool 5.

The first protruding section can be said as the first spring part. In the example shown in FIG. 22, the corrugated circumference section 120 has four first spring parts. Each first spring part has a convex shape for the side of first direction. Each first spring part does not cross the central surface CL1 of the anastomosis device 3 in the longitudinal direction. The second protruding section can be said as the second spring part. In the example shown in FIG. 22, the corrugated circumference section 120 has four second spring parts. Each second spring part has a convex shape for the side of second direction. Each second spring part does not cross the central surface CL1 of the anastomosis device 3 in the longitudinal direction. Each first spring part does not overlap with any second spring part in a direction along the first direction (in other words, when viewed from the direction perpendicular to the first direction). Each second spring part does not overlap with any first spring part in a direction along the first direction (in other words, when viewed from the direction perpendicular to the first direction). Note that in the example shown in FIG. 22, the first spring part and the second spring part are alternately arranged along the circumferential direction of the anastomosis device 3.

It is possible to say that the anastomosis device in the embodiments shown in FIG. 18 to FIG. 21, and FIG. 22 to FIG. 25 has the following configuration. That is, (A) the anastomosis device has the plurality of straight-line member which contains first straight-line like members (110A-1, 124B-12, 110B-12). (B) The anastomosis device has the plurality of spring parts that contain the first spring parts (124B-11, 120A-12, 124A-12) and the second spring parts (124A-1, 120B-1, 124B-1). The first spring part has a convex shape protruding to the first direction and the second spring part has a convex shape protruding to the second direction. (C) The plurality of straight-line members and the plurality of spring parts are alternately arranged along the circumferential direction of the anastomosis device 3. (D) The first straight-line member has the first free end engageable with the first tubular body 1 at its one end, and the second free end engageable with the second tubular body at its other end. Each straight-line member may have the first free end engageable with the first tubular body 1 at its one end and the second free end engageable with the second tubular body 2 at its other end. (E) The first straight-line member is connected to one straight-line like member (110A-11, 124A-11, 110B-11) arranged on the one side of the first straight-line member through only one spring part of the plurality of spring parts (the first spring part). Each straight-line member may be connected to one straight-line member arranged on the one side of each straight-line member through only one spring part of the plurality of spring parts. (F) The first straight-line like member is connected to another straight-line like member (110A-2, 124A-2, 110B-2) arranged on the other side of the first straight-line member through only one spring part of the plurality of spring parts (the second spring part). Each straight-line member may be connected to another straight-line member arranged on the other side of straight-line member through only one spring part of the plurality of spring parts.

Note that the anastomosis device according to the embodiment is configured by an optional biocompatible material. The anastomosis device may be configured by a bioabsorbable material.

The present invention is not limited to each of the above embodiments and each embodiment can be changed or modified appropriately in the range of the technique thought of the present invention. Also, various techniques used in each embodiment or each modification example are possible to apply to another embodiment, unless any technical contradiction is caused.

The present application is based on Japanese patent application 2015-85399 filed on April 17, 2015, and claims a priority based on the application. The disclosure thereof is incorporated herein by reference.

## Claims

1. An anastomosis device which combines a first tubular body and a second tubular body, comprising:
a corrugated circumference section having a plurality of top sections and a plurality of bottom sections, which are alternately arranged;
a top-side slender member; and
a bottom-side slender member,
wherein the top-side slender member comprises:
a first proximal end connected to one of the plurality of top sections; and
a first free end engageable with the first tubular body,
wherein the top-side slender member protrudes from the one top section for a first direction,
wherein the bottom-side slender member comprises:
a second proximal end connected to one of the plurality of bottom sections; and
a second free end engageable with the second tubular body, and
wherein the bottom-side slender member protrudes from the one bottom section for a second direction which is different from the first direction.

2. The anastomosis device according to claim 1, wherein the corrugated circumference section is configured to have a single corrugation.

3. The anastomosis device according to claim 1, wherein the corrugated circumference section is configured to have a single corrugation,
wherein the first free end has a first retaining section which is engageable with the first tubular body, and
wherein the second free end has a second retaining section which is engageable with the second tubular body.

4. The anastomosis device according to any one of claims 1 to 3, wherein the corrugated circumference section comprises:
a plurality of straight-line parts;
a plurality of first spring parts, each of which has a convex shape in the first direction; and
a plurality of second spring parts, each of which has a convex shape in the second direction, and
wherein each of the plurality of straight-line parts is connected with a corresponding one of the plurality of first spring parts and connected with a corresponding one of the plurality of second spring parts.

5. The anastomosis device according to any one of claim 1 to 4, wherein a part of the top-side slender member and a part of the corrugated circumference section are arranged in one straight line, and
wherein a part of the bottom-side slender member and a part of the corrugated circumference section are arranged in one straight line.

6. The anastomosis device according to claim 4, wherein each of the plurality of first spring parts and each of the plurality of second spring parts are arranged along a longitudinal direction of the anastomosis device so as not to overlap each other.

7. The anastomosis device according to any one of claims 1 to 6, wherein a diameter of a central section of the anastomosis device when the central section of the anastomosis device in a longitudinal direction is diameter-contracted by a loop-like wire is defined as a diameter L1,
wherein a diameter of an end section of the anastomosis device when the central section of the anastomosis device in the longitudinal direction is diameter-contracted by the loop-like wire is defined as a diameter L2, and
wherein a value when the diameter L2 is divided by the diameter L1 is 1 or more and 1.3 or less.

8. The anastomosis device according to claim 1, wherein the first free end has a first retaining section, and the second free end has a second retaining section.

9. The anastomosis device according to claim 8, wherein the first free end has a plurality of retaining sections, containing the first retaining section, and
wherein the second free end has a plurality of retaining sections, containing the second retaining section.

10. The anastomosis device according to any one of claims 1 to 9, wherein the corrugated circumference section is configured of a linear member,
wherein a width of the linear member is wider than that of the top-side slender member, and
wherein a width of the linear member is wider than that of the bottom-side slender member.

11. An anastomosis device comprising:
a corrugated circumference section having a plurality of top sections and a plurality of bottom sections, which are alternately arranged;
a plurality of top-side slender members; and
a plurality of bottom-side slender members,
wherein each of the plurality of top-side slender members has a first proximal end connected to one of the plurality of top sections, and a first free end, and
wherein each of the plurality of bottom-side slender members has a second proximal end connected to one of the plurality of bottom sections, and a second free end.
